**Europäisches Patentamt**

(19) **European Patent Office**

**Office européen des brevets**

(11) Numéro de publication : **0 149 391**
**B1**

(12)
# FASCICULE DE BREVET EUROPÉEN

(45) Date de publication du fascicule du brevet :
07.09.88

(51) Int. Cl.⁴ : **A 61 F 5/44, A 61 F 2/04,**
**A 61 M 25/02**

(21) Numéro de dépôt : **84402639.3**

(22) Date de dépôt : **18.12.84**

(54) Dispositif pour l'écoulement de l'urine des urétérostomies.

(30) Priorité : **20.12.83 FR 8320386**

(43) Date de publication de la demande :
**24.07.85 Bulletin 85/30**

(45) Mention de la délivrance du brevet :
**07.09.88 Bulletin 88/36**

(84) Etats contractants désignés :
**AT BE CH DE FR GB IT LI LU NL SE**

(56) Documents cités :
**EP-A- 0 113 489**
**DE-A- 3 140 192**
**FR-A- 2 329 295**
**FR-A- 2 511 240**
**US-A- 1 497 722**
**US-A- 2 759 477**
**US-A- 2 898 917**
**US-A- 2 900 979**
**US-A- 2 936 757**
**US-A- 3 572 340**
**US-A- 3 884 235**
**US-A- 3 927 672**
**US-A- 4 080 970**
**US-A- 4 106 507**
**US-A- 4 265 243**

(73) Titulaire : **LABORATOIRES BIOTROL S.A.**
**1, rue du Foin**
**F-75140 Paris Cedex 03 (FR)**

(72) Inventeur : **De Backer, Emile**
**6, rue François Stroobant**
**B-1060 Bruxelles (BE)**

(74) Mandataire : **Phélip, Bruno et al**
**c/o Cabinet Harlé & Phélip 21, rue de La Rochefou-**
**cauld**
**F-75009 Paris (FR)**

Jouve, 18, rue St-Denis, 75001 Paris, France

## Description

La présente invention concerne un dispositif permettant l'écoulement de l'urine des urétérostomies cutanées dans les poches de recueil utilisées habituellement par des personnes ayant subi l'abouchement d'un ou des deux uretères à la paroi abdominale lors d'une intervention chirurgicale.

Deux techniques chirurgicales sont actuellement pratiquées pour les urétérostomies.

La première technique, dite technique de Bricker, crée une jonction artificielle entre le ou les uretères à traiter et un fragment d'iléon ; le fragment d'iléon isolé est abouché à la peau pour permettre l'évacuation des urines qui y pénètrent du fait de l'anastomose de l'un ou des uretères à ce fragment. La seconde technique comporte l'abouchement direct de l'un ou des deux uretères à la peau. Chacune de ces techniques a des inconvénients. La première technique est plus compliquée ; en outre, elle peut entraîner des déséquilibres ioniques par réabsorption de certains ions au niveau de l'iléon. Lorsqu'il y a des pertes importantes de potassium, les déséquilibres organiques peuvent causer plus ou moins de dommages.

La deuxième technique d'urétérostomie cutanée est chirurgicalement plus simple mais il y a un risque important de sténose de l'abouchement de l'uretère à la peau ; on a ainsi préconisé l'introduction d'une sonde dans cet abouchement, sonde qui doit être maintenue en place par un moyen quelconque. Ces intubations sont relativement précaires et à l'origine de nombreuses difficultés.

En outre, les urines sont collectées dans des poches de recueil soit adhérant directement à la peau autour de la stomie grâce à un composé adhésif situé sur la face extérieure de la poche, soit fixées par l'intermédiaire d'une plaque portant une gomme qui adhère à la peau, plaque sur laquelle sera fixée par collage ou encliquetage la poche de recueil. Ces systèmes présentent des inconvénients non négligeables à l'usage : l'irritation de la peau due au retrait périodique des poches par mesure d'hygiène, difficulté de faire coïncider le diamètre de la stomie au niveau de la peau avec le diamètre de l'ouverture de la plaque adhésive intermédiaire ou de la poche de recueil, et surtout problèmes dus à l'agressivité de l'urine qui, lorsqu'elle vient au contact avec l'adhésif le décolle, ou fait perdre à la gomme adhésive ses qualités ou même la dissout.

Le dispositif selon l'invention, utilisable dans le cas des urétérostomies cutanées, supprime les inconvénients précédemment cités et permet d'employer la seconde technique chirurgicale, plus simple et moins préjudiciable au malade que la première. On a aussi préconisé pour les urétérostomies cutanées d'implanter chirurgicalement un dispositif d'évacuation de l'urine, comme celui décrit dans le brevet FR-A-2 511 240, composé d'un canal avec des points d'ancrage. Un tel dispositif inamovible sans nouvelle opération chirurgicale, même réalisé dans des matériaux parfaitement biocompatibles, aura des inconvénients évidents avec le temps ; par ailleurs, dans le cas où une seconde urétérostomie doit être pratiquée après la première, il faudra réopérer celle-ci pour implanter le dispositif à deux branches décrit dans cette demande.

Par ailleurs, un dispositif tel que celui décrit dans le brevet US 3 884 235 pour les iléostomies et colostomies n'est pas applicable dans le cas des urétérostomies. Si le système composé de la poche de recueil munie d'un embout est suffisamment étanche pour le recueil des matières peu fluides sortant de l'iléon, ceci ne serait pas le cas pour le recueil d'urines ; l'étanchéité n'est en effet assurée que par le contact entre l'embout et l'intérieur du conduit iléal.

Le dispositif selon l'invention est constitué :

— d'un tube creux ayant un diamètre extérieur tel que le tube puisse être introduit dans l'extrémité distale d'un uretère après son abouchement chirurgical à la paroi abdominale, de longueur comprise entre 5 et 10 cm environ et de forme éventuellement légèrement conique de façon à ce que la partie la plus étroite soit introduite facilement dans l'uretère ;

— d'une collerette ou plaque solidaire de ce tube, de forme quelconque mais avantageusement en forme de disque, portant sur la face qui sera située vers le corps du malade, un revêtement adhérant à la peau et placée de telle sorte que la longueur du tube situé entre l'extrémité pénétrant dans la stomie et la plaque soit comprise entre 30 mm et 60 mm environ. La face adhésive de la plaque est avantageusement protégée, jusqu'à l'utilisation, par un film s'enlevant facilement, tel qu'une feuille de papier.

La collerette adhésive a de préférence une dimension telle qu'elle peut recouvrir totalement l'ouverture d'introduction des poches de recueil habituelles, mais aussi éventuellement en outre la zone adhésive lorsque la poche de recueil en comportera une comme moyen de fixation au corps du malade.

Toutefois dans une autre forme d'utilisation de l'invention, le dispositif est relié à une poche de recueil de l'urine au moyen d'un tuyau souple enfilé sur le tube extérieur et la dimension de la collerette peut alors être inférieure. Dans ce cas aussi le tube extérieur peut être muni de tout moyen permettant de limiter le déplacement imprévu du tuyau sur le tube.

Le tube creux peut être divisé d'un des côtés de la plaque ou collerette en deux branches sensiblement parallèles, distantes de quelques mm, on a alors un dispositif adapté aux malades sur lesquels une double urétérostomie a été pratiquée et dont les stomies ont été placées en deux endroits de la paroi intestinale suffisamment proches pour que chaque branche soit située dans l'un des deux uretères. Dans une forme de réalisation particulière, le tube, vers l'extrémité qui n'est pas à introduire dans la stomie, est muni de perforations latérales, par lesquelles l'urine s'écoulera, au cas où une face de la poche de recueil se collerait sur l'extrémité du cathéter.

Dans une autre forme de réalisation de l'invention, cette même extrémité du tube est fermée et l'urine ne s'écoulera que par les perforations latérales dont le nombre et le diamètre seront tels qu'aucun gradient de pression ne pourra apparaître à l'intérieur du tube.

Ces dispositifs peuvent être réalisés dans des matériaux variés, on choisira de préférence le même matériau pour le tube et la collerette, mais on peut concevoir de fabriquer séparément ces deux parties pour les fixer ensuite l'une à l'autre par tout moyen connu, notamment par soudure. Les matériaux sont nécessairement biocompatibles, non toxiques et non attaqués par l'urine ; ils peuvent être rigides ou souples ; on préfère les produits souples, sans être mous, pour que le dispositif ne soit pas à l'origine de traumatismes, notamment lors de son introduction dans les uretères, ou à la suite d'un choc, pendant que le malade le porte.

On utilisera par exemple des polyoléfines comme le polyéthylène ou le polypropylène, des polyamides, des chlorures de polyvinyle, les polytétrafluoroéthylènes et le dispositif pourra être fabriqué par moulage, par injection, par rotation, par trempage ou autres, selon des méthodes bien connues de l'homme de l'art.

La substance adhésive qui se trouve sur l'une des faces de la plaque permet de fixer le dispositif sur le corps du patient, par collage sur la peau autour de la stomie ; ceci rend ce dispositif parfaitement autonome de la poche de recueil, sans pour autant présenter les inconvénients des sondes utilisées actuellement. Cette substance adhésive peut se trouver répartie sur toute la face de la collerette, mais aussi se présenter par points ou par zones. Le spécialiste pourra choisir un produit bien connu dans ce domaine, tel qu'une masse d'adhésif à base d'acrylates ou d'oxyde de Zn, qui sera appliquée en un film continu ou non de quelques microns d'épaisseur par enduction, collage, soudure ou autres, sur la face convenable de la collerette, ou encore une gomme synthétique adhésive, anallergique et stable, telle que celle décrite dans le brevet FR-A-2 479 002 qui sera appliquée en une couche mince de 0,5 à 2 mm, et de préférence 1 à 1,5 mm d'épaisseur.

Les dispositifs selon l'invention ont une longueur totale de 40 à 80 mm environ ; le tube creux a un diamètre extérieur adapté au diamètre des uretères, c'est-à-dire compris entre 2 et 4 mm, pour un adulte de taille normale. Il peut être plus petit pour convenir à des enfants, ou plus grand lorsque les uretères sont dilatées pour une raison quelconque. Le conduit intérieur a un diamètre compris entre 1 et 3 mm, de telle sorte que la paroi du tube ait une épaisseur de 0,5 à 1 mm environ. La collerette est située sur le tube à une distance de 5 à 15 mm de l'extrémité qui ne pénètre pas dans la stomie. L'épaisseur de la collerette ou plaque est de préférence inférieure à 1 mm, et elle est réalisée en un matériau qui assure à l'ensemble collerette plus adhésif une certaine souplesse afin qu'elle s'adapte bien au corps du malade autour de la stomie. Lorsque la collerette est en forme de disque, son diamètre extérieur est compris entre 20 et 40 mm, ce qui est supérieur au diamètre des ouvertures des poches de recueil classiques, et même avantageusement de 30 à 40 mm.

Un tel système d'intubation étant amovible et très manipulable peut être introduit et retiré facilement par le malade lui-même en cas de gêne ou pour le remplacer périodiquement. Par ailleurs, sa forme évite le contact de l'urine avec la peau du patient et la partie de la plaque adhérant à la peau puisque la poche de recueil de l'urine sera fixée directement ou par l'intermédiaire d'un tuyau conducteur à la face extérieure de la plaque par collage.

L'invention va être maintenant décrite plus en détail à l'aide de certains modes de réalisation choisis à titre illustratif et nullement limitatifs, en faisant notamment référence aux figures annexées :

Fig. 1 est une vue schématique en perspective d'un dispositif selon l'invention à une tubulure interne pour urétérostomie simple.

Fig. 2 et 3 sont des vues schématiques en perspective d'un dispositif selon l'invention à deux tubulures internes pour stomies sur les deux uretères et d'une variante de réalisation.

Le dispositif à une tubulure selon l'invention est constitué d'un tube creux 2 dans lequel s'écoule l'urine, et d'une collerette solidaire 1 qui délimite sur le tube une partie plus longue, avantageusement conique, plus fine à l'extrémité libre, qui sera introduite dans l'uretère par le malade lui-même, éventuellement sous surveillance médicale ; la face adhésive 3 de la collerette est située du côté de cette partie de tubulure la plus longue 4. Vers l'extrémité de la partie la plus courte 2 de la tubulure, qui se situe hors du corps du malade, se trouvent avantageusement des perforations 5 qui, dans une réalisation préférée, sont diamétralement opposées. Avant l'usage la partie adhésive 3 est recouverte d'une feuille protectrice.

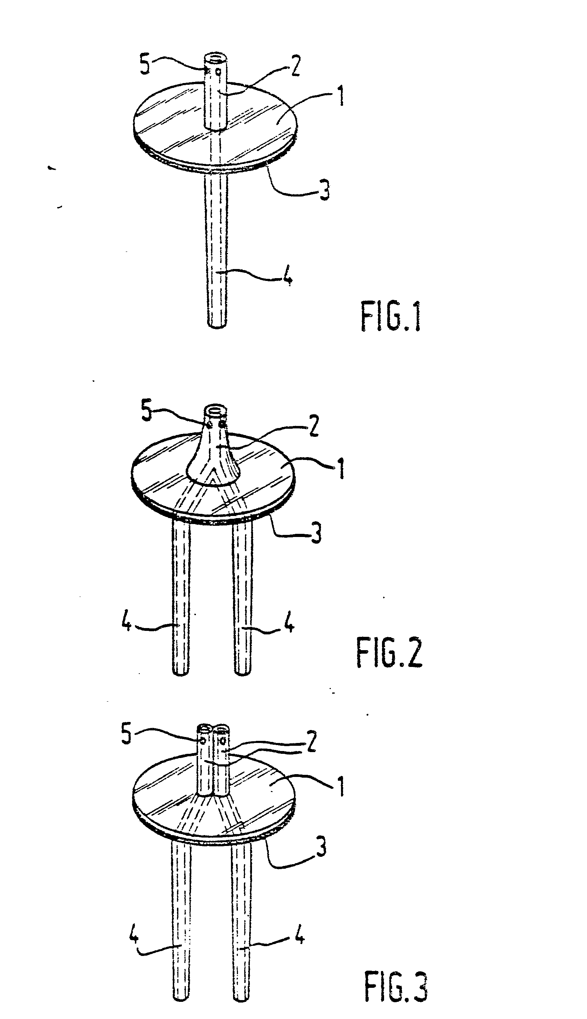

Le dispositif à deux tubulures pour double urétérostomie est composé de deux tubes 4, dont le diamètre de la section est 2 à 4 mm et qui sont parallèles et distants de 1 à 10 mm pour s'adapter à la position relative des deux stomies pratiquées sur le corps du patient ; ces deux tubulures se réunissent au niveau de la collerette en une seule de diamètre intérieur sensiblement double, et plus courte 2 par laquelle l'urine s'écoulera directement dans la poche de recueil ou dans un tuyau relié à une poche de recueil située hors de la zone péristomiale. Dans un autre mode de réalisation du dispositif de l'invention, les deux tubulures viennent simplement en contact selon une génératrice du tube creux (Fig. 3).

Dans certains dispositifs selon l'invention, les perforations 5 peuvent ne pas être présentes.

Elles sont cependant préférées et leur nombre peut être compris entre un et une dizaine, pour un diamètre inférieur au 1/10e de mm. Elles doivent être suffisantes pour assurer l'écoulement de l'urine dans la poche de recueil au cas où la face de celle-ci viendrait adhérer à l'extrémité du tube du dispositif, sans toutefois fragiliser exagérément l'extrémité dudit tube.

Pour utiliser le dispositif correspondant à l'une des formes de réalisation de l'invention, le malade, après avoir enlevé la feuille protectrice de la face adhésive de la collerette introduit la tubulure la plus longue 4 dans l'extrémité distale de son uretère, ou les tubulures dans les extrémités des uretères, jusqu'à ce que la face de la collerette 3 soit appliquée sur la peau, où elle adhère par simple pression. Il place ensuite une poche de recueil, du type de celles commercialisées, de telle sorte que la partie 2 du tube pénètre dans la poche par son ouverture prévue pour se placer face à la stomie. La poche est fixée par simple collage, comme habituellement, sur la zone péristomiale, ce qui entraîne qu'elle soit collée sur la face 1 de la collerette du dispositif et non directement sur la peau, évitant que lors de son retrait pour la vider il y ait irritation de la peau, puisque le dispositif reste en place. L'urine sort en continu de la stomie par la tubulure 4 puis se déverse dans la poche de recueil à une certaine distance de la peau, par l'extrémité du tube 1, ou par les perforations 5 ; ceci évite que l'urine ne vienne au contact des zones adhésives et de la peau.

## Exemple 1

Le dispositif présenté figure 1 a été réalisé en chlorure de polyvinyle (PVC) de faible dureté par injection.

Le disque avait 30 mm de diamètre, une épaisseur au centre de 0,5 mm et à la périphérie de 0,1 mm, la partie 2 du tube, pénétrant dans la poche de recueil, avait une longueur de 10 mm et un diamètre extérieur de 3,5 mm et celle 4, pénétrant dans la stomie, avait une longueur de 50 mm et le même diamètre. Le diamètre intérieur de ce conduit était de 2,8 mm, 2 perforations de 2 mm de diamètre ont été pratiquées à 3 mm de l'extrémité du tube, de façon diamétralement opposée.

La face 3 du disque était enduite avec une gomme synthétique adhésive classique à base de polymères et d'hydrocolloïdes, compatible avec la peau et les muqueuses. Une rondelle de papier siliconé recouvrait la gomme jusqu'à l'emploi.

## Exemple 2

Le dispositif présenté figure 2 a été réalisé, par injection, en PVC de faible dureté. Le disque 1 avait une épaisseur au centre de 0,5 mm et à la périphérie de 0,1 mm, son diamètre étant de 40 mm. Le tronçon 2, de 10 mm de long, avait un diamètre extérieur de 6 mm à son extrémité et de 10 mm au niveau du disque, alors que le diamètre intérieur du tube d'écoulement d'urine était de 4 mm ; 2 perforations, diamétralement opposées, situées à 3 mm de l'extrémité et de 3 mm de diamètre ont été pratiquées.

Les deux tubes 4, identiques, avaient 50 mm de long, 4 mm de diamètre extérieur et 2,8 mm de diamètre intérieur. Ils étaient situés à 2 mm l'un de l'autre.

La gomme synthétique adhésive compatible avec les muqueuses a été appliquée par enduction à froid. Elle était à base de polymères et hydrocolloïdes.

## Exemple 3

Le dispositif a été réalisé en chlorure de polyvinyle de faible dureté par moulage par injection. La collerette 1 avait la forme d'un rectangle de dimensions 80 mm et 100 mm de côtés, avec une épaisseur de 0,5 mm.

Le tronçon 2, de 10 mm de long et 5 mm de diamètre moyen présentait une succession de 2 nervures et 2 gorges de 1 mm de profondeur sur sa circonférence ; le tronçon 4 avait 50 mm de long et 4 mm de diamètre extérieur.

Une gomme adhésive a été appliquée sur toute la face 3 sur une épaisseur de 1 mm.

Avec ce dispositif, le recueil d'urine se fait dans une poche fixée sur la jambe de l'utilisateur. La liaison entre la poche et le dispositif est réalisée par un tuyau souple en chlorure de polyvinyle de 5 mm de diamètre intérieur, qui lors de l'usage est fixé sur le tronçon 2 du tube du dispositif.

## Revendications

1. Dispositif amovible pour l'écoulement de l'urine des urétérostomies qui se compose :
   — d'un tube creux (2), ayant un diamètre extérieur permettant son introduction dans une stomie d'uretère ;
   — d'une collerette (1) autour de ce tube, solidaire de celui-ci et située de telle sorte que la longueur du tube devant pénétrer dans la stomie soit comprise entre 30 mm et 60 mm, ladite collerette étant munie sur la face (3) qui sera en regard du corps du malade d'un produit susceptible d'adhérer à la peau.

2. Dispositif selon la revendication 1 pour les cas d'urétérostomie double, caractérisé en ce que la partie du tube creux devant pénétrer dans le corps est formé de deux branches, chacune d'entre elles étant destinée à être introduite dans une stomie.

3. Dispositif selon l'une des revendications 1 ou 2, caractérisé en ce que la collerette est de dimension telle qu'elle peut recouvrir totalement l'ouverture d'introduction des poches de recueil d'urines et, lorsque les poches en sont munies, la zone adhésive de la poche.

4. Dispositif selon l'une des revendications précédentes dans lequel la partie de tube pénétrant dans la stomie a un diamètre inférieur vers son extrémité.

5. Dispositif selon l'une quelconque des revendications précédentes, caractérisé en ce que le tube creux est muni près de son extrémité restant extérieure au corps de perforations (5) latérales.

6. Dispositif selon l'une quelconque des revendications 1, 2, 4 ou 5 caractérisé en ce que la partie restant extérieure au corps est munie de moyens de fixation pour un tuyau souple, adaptable dessus.

7. Dispositif selon l'une quelconque des revendications précédentes caractérisé en ce que la face adhésive de la collerette porte avant usage un film protecteur.


**Claims**

1. Detachable device for draining urine from ureterostomies, which is composed of

a hollow tube (2), having an external diameter enabling it to be introduced into a ureter ostomy ;

a flange (1) around this tube, firmly attached to the latter and located in such a way that the length of the tube which is to pass into the ostomy is between 30 mm and 60 mm, the said flange being provided on the face (3) which will be towards the patient's body with a product capable of adhering to the skin.

2. Device according to Claim 1 for cases of double ureterostomy, characterized in that the portion of the hollow tube which is to pass into the body is composed of two branches, each of these being designed to be introduced into an ostomy.

3. Device according to one of Claims 1 and 2, characterized in that the flange is of such a size that it can completely cover the inflow opening of bags for collecting urine and, when the bags are provided therewith, the adhesive area of the bag.

4. Device according to one of the preceding claims, in which the portion of the tube passing into the ostomy has a smaller diameter towards its end.

5. Device according to any one of the preceding claims, characterized in that the hollow tube is provided, close to its end remaining outside the body, with lateral perforations (5).

6. Device according to any one of Claims 1, 2, 4 or 5, characterized in that the portion remaining outside the body is provided with means of attachment for a flexible tube, which may be fitted thereto.

7. Device according to any one of the preceding claims, characterized in that the adhesive face of the flange bears a protective film before use.


**Patentansprüche**

1. Abnehmbare Vorrichtung zum Ableiten des Urins aus einer Ureterostomie, bestehend aus :
— einem hohlen Rohr (2) mit einem Außendurchmesser, der die Einführung des Rohrs in eine Ureterostomie erlaubt ;
— einem um dieses Rohr angeordneten Kragen (1), der mit dem Rohr fest verbunden und derart angeordnet ist, daß die Länge des Rohrs, das in die Ureterostomie eindringen soll, zwischen 30 mm und 60 mm beträgt, wobei der Kragen an der zum Körper des Kranken weisenden Fläche (3) mit einem auf der Haut haftfähigen Mittel versehen ist.

2. Vorrichtung nach Anspruch 1 für doppelte Ureterostomie, dadurch gekennzeichnet, daß der Teil des hohlen Rohrs, der in den Körper eindringen soll, aus zwei Abzweigungen besteht, von denen jede zum Einführen in eine Ureterostomie vorgesehen ist.

3. Vorrichtung nach einem der Ansprüche 1 oder 2, dadurch gekennzeichnet, daß der Kragen derart dimensioniert ist, daß er die Einführungsöffnung der Urinsammeltaschen und die Haftzone der Tasche vollständig bedecken kann, wenn die Taschen mit solcher ausgestattet sind.

4. Vorrichtung nach einem der vorhergehenden Ansprüche, bei der der in die Ureterostomie eindringende Rohrteil zu seinem Ende hin einen geringeren Durchmesser aufweist.

5. Vorrichtung nach einem der vorhergehenden Ansprüche, dadurch gekennzeichnet, daß das hohle Rohr in der Nähe seines außerhalb des Körpers bleibenden Endes mit seitlichen Perforationen (5) versehen ist.

6. Vorrichtung nach einem der Ansprüche 1, 2, 4 oder 5, dadurch gekennzeichnet, daß der außerhalb des Körpers bleibende Teil mit Befestigungsmitteln für einen biegsamen, oben anpaßbaren Schlauch versehen ist.

7. Vorrichtung nach einem der vorhergehenden Ansprüche, dadurch gekennzeichnet, daß die Haftfläche des Kragens vor Gebrauch eine Schutzfolie trägt.

FIG.1

FIG.2

FIG.3